# EUROPEAN PATENT SPECIFICATION

(11) **EP 1 237 642 B1**
(45) Date of publication and mention of the grant of the patent: **27.08.2003**
(21) Application number: 00979761.4
(22) Date of filing: 28.11.2000
(51) Int. Cl.: B01D 53/00, G07F 15/00

(54) **OXYGEN DISPENSER**
SAUERSTOFFSPENDER
DISTRIBUTEUR D'OXYGENE

(30) Priority: 30.11.1999 GB 9928221; 29.06.2000 GB 0015885
(43) Date of publication of application: 11.09.2002
(73) Proprietor: O2 Live Limited, London NW2 2NY (GB)
(72) Inventor: Simler, Dominic, Adam, London NW2 2NY (GB)
(74) Representative: Harrison Goddard Foote
(86) International application number: GB0004524
(87) International publication number: WO01039867

(56) References cited:
- DE-A- 4 012 464
- FR-A- 2 769 233
- US-A- 4 893 615
- PATENT ABSTRACTS OF JAPAN vol. 016, no. 039 (P-1305), 30 January 1992 (1992-01-30) & JP 03 245290 A (KOMATSU RAITO SEISAKUSHO:KK), 31 October 1991 (1991-10-31)

## Description

### Field of the Invention

The present invention relates to an oxygen dispenser and more particularly although not necessarily exclusively to oxygen vending systems.

### Background to the Invention

The benefits of inhaling substantially pure oxygen to compensate for hypoxia are becoming increasingly broadly appreciated. Most commonly, individuals suffer from hypoxia following high levels of exertion in a relatively poorly ventilated environment. Such might be the case in a bar or night club particularly but may arise in a health club or other environment. Indeed, in cities where there is a very high level of atmospheric polution from, for example, car exhausts access to substantially pure oxygen or oxygen-enriched air may be an important factor in maintenance of good health.

Existing sources of substantially pure oxygen supply are conventionally simple oxygen cylinder based systems which have a manual valve for dispensing of the oxygen by an attendant or are adapted for self-service and have temporary storage of oxygen in a container intermediate the cylinder and mouth piece. These systems are, however, impractical for widespread usage and it is, of course, impractical to dispense individual oxygen cylinders to users.

It is a general objective of the present invention to provide an oxygen dispensing system that is versatile, being suitable for installation in any of a wide range of different environments and usable in an efficient and economic manner.

### Summary of the Invention

According to a first aspect of the present invention there is provided an oxygen dispenser for dispensing substantially pure oxygen or highly oxygen enriched air, which dispenser comprises a housing accommodating an oxygen concentrator in fluid communication with an air inlet of the housing and a dispenser outlet of the housing via pipework, a flow control valve to control flow of oxygen or oxygen-enriched air to the dispenser outlet, the flow control valve being under the control of a controller, the dispenser unit further having a card reader for a mag-stripe, swipe card, smart card or similar or a receiver for credit tokens or other credit data input means whereby credit units may be input to the dispenser controller to dispense oxygen or oxygen enriched air.

The valve is preferably a solenoid-operated valve and it may suitably be operated by the controller to dispense a predetermined volume of oxygen or oxygen enriched air for each unit of credit input by card or token or other means, which may include currency coins or notes.

The card is preferably a coded magnetic stripe (mag-stripe) card which may have the one or more credits on the card deleted following use.

In addition to having the automated control of the valve to dispense oxygen or oxygen enriched air in response to the credit data input by the card, tokens or other means, the unit preferably also has means operable by the user to alter flow rate and/or to switch off flow of oxygen or oxygen-enriched air. The dispenser unit suitably has a dispenser outlet to which a nasal or other dispensing cannula or dispensing mask may be fitted for each dispensing operation and which may be automatically cut off from further oxygen or oxygen-enriched air dispensing supply when the mask or cannula is detached from the dispenser outlet.

In contrast to incorporating one or more oxygen cylinders, the dispenser incorporates an oxygen concentrator, providing a substantially inexhaustible supply of oxygen-enriched air. An oxygen concentrator in its broadest sense is any device that concentrates the oxygen already present in air.

Most oxygen concentrators use PSA (Pressure Swing Adsorption) technology to concentrate the oxygen. PSA consists of a process where a gas is fed at an elevated pressure to a vessel containing an adsorbent matrix. The adsorbent matrix selectively adsorbs one or more of the non oxygen gas components such as Nitrogen and carbon dioxide. Thus, the produced gas is enriched in the oxygen and any other components that have had least adsorption. The adsorbent bed is regenerated by: 1. reducing the pressure in the vessel and 2. flowing some high purity gas through the adsorbent particles. At least two adsorbent beds are used so that continuous flow of the enriched gas can be obtained. When one vessel is adsorbing gas, the adsorbent in the other vessel is being regenerated. Typical adsorbent materials which are used comprise carbon molecular sieves, zeolite molecular sieves, activated carbon, silica gel, and activated alumina.

The oxygen dispenser is particularly efficient when the concentrator is operated continuously. This may have benefits in terms of the useable life of the oxygen concentrator or its maintenance intervals and is particularly useful in avoiding delay in supply of highly oxygen enriched air to the user. Accordingly, the dispenser is adapted to have the concentrator running continuously and configured to disperse the oxygen or oxygen-enriched air into the surrounding atmosphere when the flow control valve is not at its setting to direct flow of the oxygen or oxygen-enriched air to the dispenser outlet.

Suitably the solenoid valve is adapted to selectively alternate between supply to the dispenser outlet or to a venting outlet at a remote location of the dispenser housing away from the dispenser outlet.

The dispenser preferably has an extractor fan to expell air from the dispenser housing.

Preferably the venting outlet has an extractor fan to expell and disperse the oxygen or oxygen-enriched air rapidly.

Alternatively or preferably additionally, the dispenser is suitably adapted so that the oxygen or oxygen-enriched air is re-combined with the oxygen-depleted air from the oxygen concentrator as it is expelled from, or preferably prior to being expelled from, the dispenser housing. Such measures prevent any risk whatsoever of accumulation of oxygen within or in the vicinity of the dispenser other than the harmless small volumes held in the concentrator and associated pipework. There is, therefore no fire hazard and, indeed, the system is far safer than the existing systems that rely on use of pressurised oxygen cylinders.

By way of further improvement, the dispenser housing suitably has an air filtration element covering the air inlet to the housing and which is externally accessible to enable easy replacement. This will facilitate maintenance of the apparatus, reducing the need for internal servicing and is particularly valuable for environments with high levels of atmospheric polution.

For user comfort and to extend the benefits of use of the apparatus it is preferably adapted to introduce fragrance into the oxygen or oxygen-enriched air that it dispenses. Aromatherapy oils or other sources of fragrance may conveniently be introduced by coupling a vessel, such as, for example, a tube/cartridge, containing a selected oil or other source of fragrance into the air line of the nasal cannula or mask that is coupled, in use, to the dispenser outlet.

In an alternative embodiment, the apparatus may be adapted to introduce fragrance into the airline upstream of the dispenser outlet within the housing. To this end, one or more fragrance holding vessels may be coupled to the pipework leading to the dispenser outlet from the oxygen concentrator and where there are several such vessels with different fragrances there is suitably a means of switching between them. This arrangement may be particularly suitable where the apparatus is to be used in a domestic or other environment where the facility for rapid change over between a wide range of different fragrances is not of such great importance.

Turning to a second aspect of the present invention there is provided an oxygen dispenser for dispensing substantially pure oxygen or highly oxygen-enriched air, which dispenser comprises a housing accommodating an oxygen concentrator in fluid communication with an air inlet of the housing and a dispenser outlet of the housing via pipework, a flow control valve to control flow of oxygen or oxygen-enriched air to the dispenser outlet, the flow control being under the control of a controller and wherein the valve is a solenoid-operated valve which is operated by the controller to dispense a volume of oxygen or oxygen enriched air in response to activation of a switch means. The swich means may be a manually operated electrical switch or may be an electrical switch that is automatically activated by coupling a dispensing cannular or dispensing mask or the like to the dispenser outlet and suitably deactivated by uncoupling of the dispensing cannular mask or the like from the outlet.

The volume of oxygen dispensed is suitably a predetermined volume and may be controlled by dispensing at a known rate (which may be fixed at the outset or variable but monitored) for a controlled period of time. The controller accordingly preferably has a timer for timing the duration of a dispensing session.

Preferably the oxygen dispenser of the invention further comprises one or more flow sensors to sense the rate of dispensing flow of oxygen or oxygen enriched air and being operatively linked to the controller.

Preferably the oxygen dispenser of the invention futher comprises one or more oxygen level sensors to sense the level of oxygen or oxygen enriched air being dispensed by the dispenser and/or the level of oxygen in the air being drawn into the oxygen concentrator, the oxygen level sensor(s) being operatively linked to the controller.

Suitably the or each oxygen level sensor forms part of a negative feedback loop with the controller. The signals from the oxygen level sensor(s) may be processed by the controller to dictate opening and or closure of the control valve to facilitate achievement and/or maintenance of a desired level of oxygen.

### Brief Description of the Drawings

A preferred embodiment of the present invention will now be more particularly described, by way of example, with reference to the accompanying drawings, wherein:

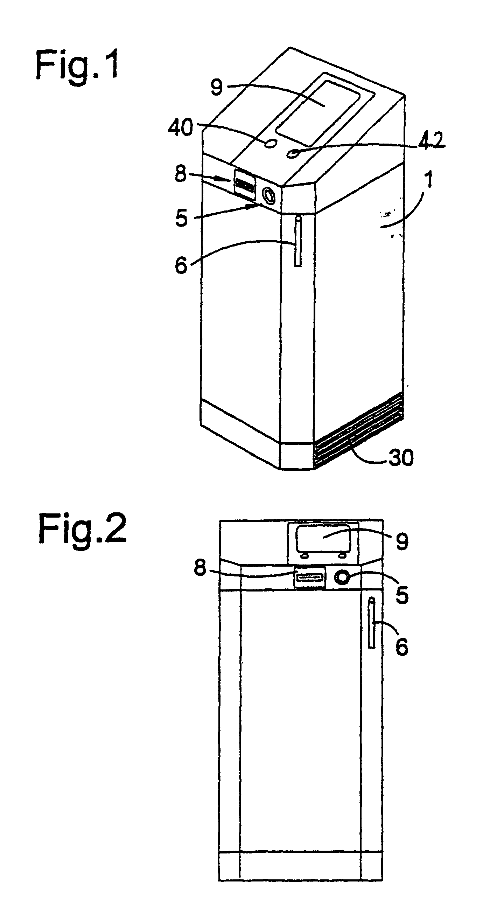

Figures 1 to 4 are, respectively, a perspective view, a front elevation view, a rear elevation view and a side part sectional view of an oxygen dispenser embodying the invention;
Figure 5 is a schematic diagram illustrating components of a conventional oxygen concentrator; and
Figure 6 is a drawing of a fragrance-containing adaptor.

### Description of the preferred embodiment

Referring to the Figures, the oxygen dispenser comprises a floor standing steel cabinet 1 housing an oxygen concentrator module 2 which is positioned within the cabinet 1 with its air inlet 50 exposed to receive air flow from a corresponding air inlet 3, in the cabinet 1. In use the oxygen concentrator is powered to operate substantially continuously.

The oxygen enriched air outlet 15 of the oxygen concentrator 2 has delivery pipework 4 coupled to it and extending upwardly towards a dispensing outlet 5 of the dispenser unit.

Part way along the length of the pipework 4 is a solenoid-operated-valve 21 that is adapted to open or close the pipework 4 to permit or stop flow of the oxygen enriched air to the dispenser outlet 5. When the solenoid-operated valve 21 stops flow to the dispenser outlet 5 it instead diverts it via a discharge pipe 60 to an outlet vent 30 that vents to atmosphere. The expulsion of the oxygen enriched air is assisted by an extractor fan 31 at or near the outlet vent 30. This fan also expels the de-oxygenated air discharged from the concentrator 2 along with the oxygen enriched air preventing pressure build up within the housing 1, assisting operation of the concentrator 2 and preventing oxygen accumulation in the housing 1. Ducting may be provided extending from the deoxygenated air outlet of the compressor 2 to near the extractor fan at the housing outlet vent 30 if necessary to prevent return of the deoxygenated air to the inlet 50 of the compressor and disruption of intake of fresh air. Alternatively or additionally, and as illustrated, the air inlet 50 of the compressor may be in exclusive fluid communication with the air inlet 3 of the housing 1 via an intake duct 49.

Further along the pipe 4 before the dispenser outlet 5 the pipework 4 enters a flow meter section 6 that is externally manually adjustable by the user to control the rate of flow of oxygen-enriched air during use.

The dispenser outlet 5 comprises a socket to which a nasal cannula or tube leading to an oxygen mask is detachably coupled, in use.

This dispenser outlet 5 is adapted to automatically cut off flow of the oxygen enriched air when the cannula/oxygen mask tube is detached from the dispenser outlet 5.

Delivery of oxygen enriched air from the oxygen concentrator 2 to the dispenser outlet 5 is initiated by setting of the solenoid valve 21 to its dispensing position by a control signal from an electronic controller when the electronic controller of the unit registers that one or more appropriate credits have been entered into the dispenser by the user. This is suitably done by inserting a swipe or mag-stripe card into a card reader 8 in the user interface at the upper front of the cabinet. Credit data may also be input by cursor keys, a numeric or alphanumeric keypad 20. Such data may involve a unique user code. A small video display 9 confirms to the user that he has properly supplied the required credit(s) and may indicate how many credits he has remaining and/or the remaining volume or duration of oxygen-enriched air supply that he may receive.

The controller comprises, in the preferred embodiment, a microprocessor linked to sensors. The controller having confirmed that there is a supply of oxygen at a suitable pressure will select an appropriate dispenser outlet 5 if there are several, flash an adjacent indicator, and on confirmation by the user that he has connected himself to the system and is ready, by the user, for example, pressing a "START' button 40 that is linked to the controller, the controllers will then send a control signal to the solenoid valve 21 to set it to deliver a flow of oxygen, for example at four litres/minute via the solenoid-operated valve 21 for a pre-determined time period or as required by the user.

Termination of flow by the solenoid valve 21 may be by the user pressing a "STOP" button 42 on the control panel or may be by the controller in response to expiry of an allowed time period for example, or may occur automatically if the user detaches their oxygen delivery tube. In one important embodiment the oxygen delivery tube that extends from the dispenser outlet 5 and to which the user's nasal cannula or oxygen mask is attached comprises an in-built retractable hose that is spring loaded and automatically retracts when not in use and thereby automatically trips a switch to switch off the oxygen supply.

For medical purposes, the swipe card may be encoded with data or signals that correspond to the medical requirements of the individual. This may restrict the duration or rate of supply of oxygen enriched air or possibly enhance it. The card may be of so-called smart card type and capable of logging details such as the parameters of each session of use of the dispenser so that the use and more particularly the nature of the use may be monitored over time. The dispenser cabinet 1 may also be adapted to enable downloading of information from the dispenser to an external computer to enable monitoring of use of the system for any of a wide variety of reasons. To this end a serial dataport or the like is suitably provided at the rear of the cabinet 1.

The normal rate of delivery of oxygen or oxygen enriched air is generally four litres per minute and suitably for a duration of a few minutes. The user may manually adjust flow via the flow meter 6 or the flow may be adjusted for him/her automatically in accordance with any details pre-programmed onto his/her card that is inserted in the card reader 8.

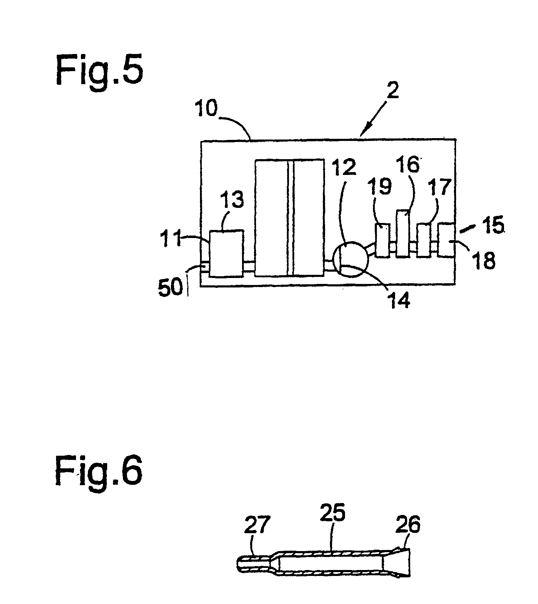

With reference to Figure 5, the oxygen concentrator module comprises an inner casing 10 within which is housed a compressor 11 to draw in air through the housing air inlet 3 and through intake duct 49 to compressor module air inlet 50 and supply it to an oxygen accumulator 12 via sieve beds 13 and filters 14 for particulates. The oxygen enriched air or substantially pure oxygen in the accumulator is then delivered to an outlet 15 of the concentrator 2 via a bacterial filter 16, flow meter 17 and check valve 18. A pressure regulator 19 at the oxygen accumulator 12 maintains the gas within the appropriate pressure levels.

These components of the concentrator 2 may be assembled within the cabinet 1 without use of a separate inner casing 10.

For ease of maintenance the housing air inlet 3 suitably has a mesh grille that accommodates behind it a pad of filter foam to trap airborne particulates, the pad being easily externally accessible upon moving the grille to enable the filter foam pad to be cleaned and replaced or substituted with a fresh filter foam pad.

For multiple users of the oxygen dispenser it may be desirable to use, for example, twin concentrators 2 of 5 litres per minute maximum output to serve two or three dispenser outlets in a single dispenser unit 1. The maximum delivery rate may of course, be substantially greater than 5 litres (eg 10 or more litres per minute), subject to the limitations of the concentrator 2 that is used and the desired energy economy of the system.

Preferably the oxygen dispenser of the invention further comprises one or more flow sensors 70 to sense the rate of dispensing flow of oxygen or oxygen enriched air and being operatively linked to the controller. It suitably also has one or more oxygen level sensors 80a, 80b, to sense the level of oxygen in the oxygen or oxygen enriched air being dispensed by the dispenser and/or the level of oxygen in the air being drawn into the oxygen concentrator, the oxygen level sensor(s) being operatively linked to the controller.

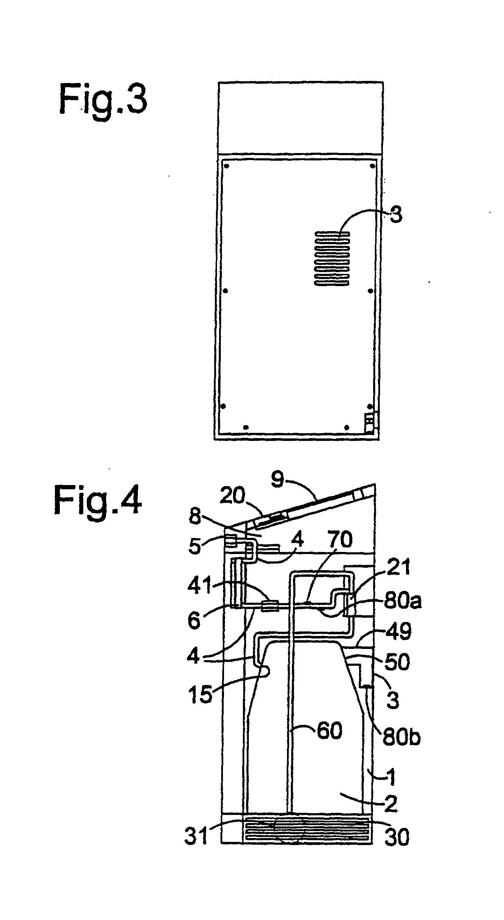

A further refinement to the system figuratively illustrated in Figure 4 is the incorporation of delivery of fragrance chemical into the supply of oxygen or oxygen enriched air to add the benefits of aromatherapy to the existing benefits of the invention.

In the illustrated embodiment the fragrance chemicals are held in a vessel or group of vessels 41. The vessel 41, or one of the vessels 41 is, at least, in use, coupled to the pipework 4 leading from the oxygen concentrator outlet 15 to the dispenser outlet 5 and within the cabinet 1. If there are several vessels 41 these suitably each contain a different fragrance chemical and may be manually or automatically switched between.

An alternative arrangement for fragrance delivery involves coupling a short tubular adaptor section 25 as illustrated in Figure 6 into the airline tubing of the mask or nasal cannula extending from the dispenser outlet 5, external to the cabinet 1, the fragrance chemical being coated on the interior of the tubular section and hence exposed to the stream of oxygen enriched air. A range of interchangeable adaptor sections each containing a different aroma may be provided. Each tubular adaptor section 25 push fits or screw fits at each end to couple to the outlet 5 at one end 26 and to the mask or nasal cannula at the other end 27.

In a further preferred embodiment the user may be provided with a mask to receive a "jet" of oxygen from the dispenser and where the mask has an in-built facility to receive a fragrance chemical for scenting the oxygen. This may comprise, for example, a slot to receive a fragrance cartridge. Alternatively, for example, a fragrance catridge may be plugged into the oxygen inlet "mouthpiece" of the mask.

Although in the above description we have focussed upon the use of a "single man" dispenser. The dispenser may be assembled as a multi user system with, for example, two conventional oxygen concentrators housed together and supplying four oxygen outlets between them via a simple manifold. In a more highly developed system, a plurality of satelite dispensing stations, e.g. sixteen, may be linked to a common centralised oxygen concentrator/controller station. Oxygen, or highly oxygen enriched air, from the centralised station may be supplied to each of the satelite stations on demand. Each satelite station may suitably comprise a socket for connecting to the user's nasal cannula or oxygen mask, the socket being supplied with oxygen from the centralised station once the user plugs his/her cannula or mask into the socket and suitably also once he/she has inserted his/her swipe card or other means of credit into a credit receiving facility at the satellite station. For this purpose each satellite station suitably has a card reader with an associated microswitch to switch open a solenoid valve for supply of oxygen enriched air to that station from the centralised station. This distribution system may, for example, be used in a gym to service users who are also using or at individual items of gym equipment enabling them to take full benefit of the enhanced oxygen to optimise their work-out and their recovery from the exertion of the work-out.

## Claims

1. An oxygen dispenser for dispensing substantially pure oxygen or oxygen-enriched air, which dispenser comprises a housing (1) accommodating an oxygen concentrator (2) in fluid communication with an air inlet of (3) the housing and a dispenser outlet (5) of the housing (1) via pipework (4), a flow control valve (21) to control flow of oxygen or oxygen-enriched air to the dispenser outlet (5), the flow control valve (21) being under the control of a controller, the dispenser being adapted to have the concentrator running continuously and configured to disperse the oxygen or oxygen-enriched air into the surrounding atmosphere when the flow control valve (21) is not at its setting to direct flow of the oxygen or oxygen-enriched air to the dispenser outlet (5), the dispenser unit further having a card reader (8) for a mag-stripe, swipe card or smart card or a receiver for credit tokens, currency coins or notes or other credit data input means to enable credit units to be input to the dispenser controller to dispense oxygen or oxygen-enriched air.

2. An oxygen dispenser as claimed in claim 1, wherein the valve (21) is a solenoid-operated valve.

3. An oxygen dispenser as claimed in claim 2 wherein the solenoid valve (21) is adapted to selectively alternate between supply to the dispenser outlet (5) or to a venting outlet (30) at a remote location of the dispenser housing (1) away from the dispenser outlet.

4. An oxygen dispenser as claimed in any of the preceding claims, wherein the valve (21) may be operated by the controller to dispense a predetermined volume of oxygen or oxygen-enriched air for each unit of credit input.

5. An oxygen dispenser as claimed in any of the preceding claims, wherein the dispenser has a card reader for a coded magnetic card.

6. An oxygen dispenser as claimed in claim 5, wherein the dispenser is adapted so that deletes the one or more credits on the card following use.

7. An oxygen dispenser as claimed in any of the preceding claims, wherein in addition to having the automated control of the valve (21) to dispense oxygen or oxygen-enriched air in response to the credit data input means, the dispenser also has means (20) operable by the user to alter rate of flow and/or to switch off flow of oxygen or oxygen-enriched air.

8. An oxygen dispenser as claimed in any of the preceding claims, wherein the dispenser unit has a dispenser outlet (5) to which a nasal or other dispensing cannula or dispensing mask may be fitted for each dispensing operation.

9. An oxygen dispenser as claimed in claim 8, wherein the dispenser is adapted so that the dispenser outlet (5) is automatically cut off from further oxygen or oxygen-enriched air dispensing supply when the mask or cannula is detached from the dispenser outlet (5).

10. An oxygen dispenser as claimed in any of the preceding claims, wherein the dispenser has an extractor fan (31) to expel air from the dispenser housing (1).

11. An oxygen dispenser as claimed in claim 10, wherein the venting outlet (30) has said extractor fan (31) to expel and disperse the oxygen or oxygen-enriched air.

12. An oxygen dispenser as claimed in any of the preceding claims, wherein the dispenser is adapted so that the oxygen or oxygen-enriched air is re-combined with the oxygen-depleted air from the oxygen concentrator as it is expelled from, or prior to being expelled from, the dispenser housing (1).

13. An oxygen dispenser as claimed in any of the preceding claims, wherein the dispenser housing (1) has an air filtration element covering the air inlet (3) to the housing and which is externally accessible to enable easy replacement.

14. An oxygen dispenser as claimed in any of the preceding claims, wherein it is adapted, in use, to introduce fragrance into the oxygen or oxygen-enriched air that it dispenses.

15. An oxygen dispenser as claimed in claim 14, wherein a fragrance holding vessel (41) is provided which couples externally to the dispenser outlet (5) or internally to the pipework (4) leading to the dispenser outlet (15), in use.

16. An oxygen dispenser as claimed in claim 15, wherein there are a plurality of fragrance holding vessels (41), each vessel holding a respective fragrance, and wherein there is further provided means of switching between the vessels.

17. An oxygen dispenser as claimed in claim 14, wherein the or each fragrance holding vessel (41) detachably couples in use to the dispenser outlet (5).

## Patentansprüche

1. Sauerstoffspender zur Ausgabe von im wesentlichen reinem Sauerstoff oder sauerstoffangereicherter Luft, mit einem Gehäuse (1), welches einen Sauerstoffkonzentrator (2) enthält, der in Strömungsverbindung mit einem Lufteinlaß (3) des Gehäuses und einem Spenderauslaß (5) des Gehäuses (1) über Rohrleitungen (4) steht, einem Strömungs-Regelventil (21) zum Regeln der Strömung des Sauerstoffs oder der sauerstoffangereicherten Luft zum Spenderauslaß (5), wobei das Strömungs-Regelventil (21) durch einen Regler betätigt wird, wobei der Spender für den kontinuierlichen Betrieb des Konzentrators ausgelegt und zum Verteilen des Sauerstoffs oder der sauerstoffangereicherten Luft in die umgebende Atmosphäre eingerichtet ist, wenn das Strömungs-Regelventil (21) sich nicht in seiner Stellung befindet, um den Strom von Sauerstoff oder sauerstoffangereicherter Luft zum Spenderauslaß (5) zu leiten, wobei die Spendereinheit ferner ein Kartenlesegerät (8) für eine Magnetstreifenkarte oder Smart-Card oder eine Aufnahmevorrichtung für Wertmünzen, Währungsmünzen, Banknoten oder andere Kreditdaten-Eingabemittel aufweist, um die Eingabe der Krediteinheiten in den Spenderregler zu ermöglichen, um Sauerstoff oder sauerstoffangereicherte Luft auszugeben.

2. Sauerstoffspender nach Anspruch 1, wobei das Ventil (21) ein magnetbetriebenes Ventil ist.

3. Sauerstoffspender nach Anspruch 2, wobei das Magnetventil (21) dazu ausgebildet ist, wahlweise zwischen Zufuhr zum Spenderauslaß (5) oder zu einem Entlüftungsauslaß (30) an einer abgelegenen Stelle des Spendergehäuses (1) entfernt von dem Spenderauslaß umzuschalten.

4. Sauerstoffspender nach einem der vorangehenden Ansprüche, wobei das Ventil (21) durch den Regler betätigt werden kann, um ein vorbestimmtes Volumen des Sauerstoffs oder der sauerstoffangereicherten Luft für jede Einheit der Krediteingabe auszugeben.

5. Sauerstoffspender nach einem der vorangehenden Ansprüche, wobei der Spender ein Kartenlesegerät für eine kodierte Mangetstreifenkarte aufweist.

6. Sauerstoffspender nach Anspruch 5, wobei der Spender derart ausgebildet ist, daß er der Benutzung folgend eine oder mehrere Krediteinheiten auf der Karte löscht.

7. Sauerstoffspender nach einem der vorangehenden Ansprüche, wobei zusätzlich zur automatischen Regelung des Ventils (21) zur Ausgabe von Sauerstoff oder sauerstoffangereichterter Luft in Abhängigkeit von den eingegebenen Kreditkartenmitteln der Spender ebenfalls Mittel (20) aufweist, die durch den Benutzer betätigbar sind, um die Strömungsrate zu verändern und/oder das Ausströmen von Sauerstoff oder sauerstoffangereicherter Luft auszuschalten.

8. Sauerstoffspender nach einem der vorangehenden Ansprüche, wobei die Spendereinheit einen Spenderauslaß (5) aufweist, an den Nasenröhrchen oder anderes Ausgaberöhrchen oder eine Ausgabemaske für jeden Ausgabevorgang angebracht werden kann.

9. Sauerstoffspender nach Anspruch 8, wobei der Spender dazu ausgebildet ist, daß der Spenderauslaß (5) automatisch von der Versorgung mit weiterem Sauerstoff oder sauerstoffangereicherter Luft abgeschnitten wird, wenn die Maske oder das Röhrchen von dem Spenderauslaß (5) entfernt wird.

10. Sauerstoffspender nach einem der vorangehenden Ansprüche, wobei der Spender einen Sauglüfter (31) zum Ausstoßen von Luft aus dem Spendergehäuse (1) aufweist.

11. Sauerstoffspender nach Anspruch 10, wobei der Lüftungsauslaß (30) den genannten Sauglüfter (31) aufweist, um Sauerstoff oder sauerstoffangereicherte Luft auszustoßen und zu verteilen.

12. Sauerstoffspender nach einem der vorangehenden Ansprüche, wobei der Spender dazu ausgebildet ist, daß der Sauerstoff oder die sauerstoffangereicherte Luft mit sauerstoffabgereicherter Luft von dem Sauerstoffkonzentrator wiedervereinigt wird, wenn oder bevor sie aus dem Spendergehäuse (1) ausgestoßen wird.

13. Sauerstoffspender nach einem der vorangehenden Ansprüche, wobei das Spendergehäuse (1) ein Luftfilter-Element aufweist, welches den Lufteinlaß (3) in das Gehäuse abdeckt und von außen zugänglich ist, um einen einfachen Austausch zu ermöglichen.

14. Sauerstoffspender nach einem der vorangehenden Ansprüche, wobei er dazu ausgebildet ist, bei der Verwendung einen Duft in den Sauerstoff oder die sauerstoffangereicherte Luft, die er ausstößt, einzuleiten.

15. Sauerstoffspender nach Anspruch 14, wobei ein Duftstoff enthaltendes Gefäß (41) vorgesehen ist, welches extern mit dem Spenderauslaß (5) oder intern mit den im Betrieb zum Spenderauslaß (5) führenden Rohrleitungen (4) verbunden ist.

16. Sauerstoffspender nach Anspruch 15, wobei eine Mehrzahl Duftstoff enthaltender Gefäße (41) vorgesehen ist, wobei jedes Gefäß einen entsprechenden Duftstoff enthält und wobei ferner Mittel zum Umschalten zwischen den Gefäßen vorgesehen sind.

17. Sauerstoffspender nach Anspruch 14, wobei das oder jedes Duftstoff enthaltende Gefäß (41) im Betrieb lösbar mit dem Spenderauslaß (5) verbunden ist.

## Revendications

1. Distributeur d'oxygène destiné à distribuer de l'oxygène sensiblement pur ou de l'air enrichi en oxygène, lequel distributeur comprend un logement (1) logeant un concentrateur (2) d'oxygène en communication liquide avec une entrée d'air (3) du logement et un refoulement de distribution (5) du logement (1) par l'intermédiaire d'une tuyauterie (4) ; un robinet de réglage (21) pour réguler l'écoulement d'oxygène ou d'air enrichi en oxygène vers la sortie de distribution, le robinet de réglage (21) étant sous la commande d'un contrôleur, le distributeur étant adapté pour avoir le concentrateur fonctionnant de manière continue et configuré pour disperser l'oxygène ou l'air enrichi en oxygène dans l'atmosphère environnante quand le robinet de réglage (21) n'est pas à son réglage pour diriger l'écoulement d'oxygène ou d'air enrichi en oxygène vers la sortie de distribution (5), l'unité de distributeur présentant en outre un lecteur de carte (8) pour une carte magnétique, une carte à fente ou une carte à puce ou un récepteur pour jetons de crédit, pièces de monnaies ou billets ou d'autres moyens d'entrée de données de crédit pour permettre aux unités de crédit d'être saisies vers le contrôleur du distributeur afin de distribuer l'oxygène ou l'air enrichi d'oxygène.

2. Distributeur d'oxygène selon la revendication 1, dans lequel le robinet (21) est une électrovanne.

3. Distributeur d'oxygène selon la revendication 2, dans lequel l'électrovanne (21) est adaptée pour alterner sélectivement entre l'alimentation vers la sortie de distribution (5) ou vers un orifice de purge (30) au niveau d'un emplacement éloigné du logement du distributeur (1) depuis la sortie de distribution.

4. Distributeur d'oxygène selon l'une quelconque des revendications précédentes, dans lequel le robinet (21) peut être actionné par le contrôleur pour distribuer un volume prédéterminé d'oxygène ou d'air enrichi en oxygène pour chaque unité de crédit.

5. Distributeur d'oxygène selon l'une quelconque des revendications précédentes, dans lequel le distributeur présente un lecteur de carte pour une carte magnétique codée.

6. Distributeur d'oxygène selon la revendication 5, dans lequel le distributeur est adapté afin qu'il efface le ou les crédits sur la carte après utilisation.

7. Distributeur d'oxygène selon l'une quelconque des revendications précédentes, dans lequel en plus de présenter la commande automatisée du robinet (21) afin de distribuer l'oxygène ou l'air enrichi en oxygène en réponse aux moyens d'entrée de données de crédit, le distributeur présente également des moyens (20) actionnables par l'utilisateur pour modifier la vitesse d'écoulement et/ou pour arrêter l'écoulement d'oxygène ou d'air enrichi en oxygène.

8. Distributeur d'oxygène selon l'une quelconque des revendications précédentes, dans lequel l'unité de distributeur présente une sortie de distribution (5) à laquelle une canule nasale ou autre canule de distribution ou un masque de distribution peut être adapté pour chaque opération de distribution.

9. Distributeur d'oxygène selon la revendication 8, dans lequel le distributeur est adapté de façon que la sortie de distributeur (5) soit automatiquement isolé d'une nouvelle alimentation en oxygène ou en air enrichi en oxygène quand le masque ou la canule est détaché de la sortie de distribution (5).

10. Distributeur d'oxygène selon l'une quelconque des revendications précédentes, dans lequel le distributeur présente un ventilateur d'extraction (31) pour expulser l'air du logement du distributeur (1).

11. Distributeur d'oxygène selon la revendication 10, dans lequel l'orifice de purge (30) présente ledit ventilateur d'extraction (31) afin d'expulser et de disperser l'oxygène et l'air enrichi en oxygène.

12. Distributeur d'oxygène selon l'une quelconque des revendications précédentes, dans lequel le distributeur est adapté afin que l'oxygène ou l'air enrichi en oxygène se recombine avec l'air appauvri en oxygène depuis le concentrateur d'oxygène alors qu'il est expulsé, ou avant d'être expulsé du logement du distributeur (1).

13. Distributeur d'oxygène selon l'une quelconque des revendications précédentes, dans lequel le logement de distributeur (1) présente un élément de filtration d'air couvrant l'entrée d'air (3) vers le logement et qui est accessible extérieurement afin de permettre un remplacement aisé.

14. Distributeur d'oxygène selon l'une quelconque des revendications précédentes, dans lequel il est adapté, en utilisation, pour introduire une fragrance dans l'oxygène ou l'air enrichi d'oxygène qu'il distribue.

15. Distributeur d'oxygène selon la revendication 14, dans lequel une cuve de retenue de fragrance (11) est proposée qui s'accouple extérieurement à la sortie de distribution (5) ou intérieurement à la tuyauterie (4) conduisant à la sortie de distribution (5), en utilisation.

16. Distributeur d'oxygène selon la revendication 15, dans lequel il existe une pluralité de cuves de retenue de fragrance (41), chaque cuve retenant une fragrance respective et dans lequel sont en outre proposés des moyens de commutation entre les cuves.

17. Distributeur d'oxygène selon la revendication 14, dans lequel la ou chaque cuve de retenue de fragrance (41) s'accouple de manière détachable en utilisation à la sortie de distribution (5).
